(19) **Europäisches Patentamt**
**European Patent Office**
**Office européen des brevets**

(11) **EP 2 008 591 B1**

(12) **EUROPEAN PATENT SPECIFICATION**

(45) Date of publication and mention
of the grant of the patent:
**09.09.2015 Bulletin 2015/37**

(51) Int Cl.:
**A61B 8/08** *(2006.01)* **A61B 8/12** *(2006.01)*

(21) Application number: **07741166.8**

(86) International application number:
**PCT/JP2007/057730**

(22) Date of filing: **06.04.2007**

(87) International publication number:
**WO 2007/116957 (18.10.2007 Gazette 2007/42)**

(54) **ULTRASONIC PROBE AND ULTRASONOGRAPH**

ULTRASCHALLSONDE UND SONOGRAFIEGERÄT

SONDE A ULTRASONS ET ECHOGRAPHE

(84) Designated Contracting States:
**DE FR GB IT NL**

(30) Priority: **07.04.2006 JP 2006105916**

(43) Date of publication of application:
**31.12.2008 Bulletin 2009/01**

(73) Proprietor: **Hitachi Medical Corporation
Tokyo 101-0021 (JP)**

(72) Inventor: **MATSUMURA, Takeshi
Tokyo 101-0021 (JP)**

(74) Representative: **MERH-IP
Matias Erny Reichl Hoffmann
Paul-Heyse-Strasse 29
80336 München (DE)**

(56) References cited:
**EP-A1- 1 629 777        WO-A1-2004/105615
JP-A- 02 154 131        JP-A- 08 112 280
JP-A- 08 322 842        JP-A- 61 107 774
JP-A- 2005 021 710      JP-A- 2005 066 041
JP-A- 2005 144 155      JP-A- 2007 014 424
US-A- 5 265 612         US-A1- 2004 210 136**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Background

The present invention relates to an ultrasonic probe and ultrasonic diagnostic apparatus for displaying a tomographic image and an elastic image that presents hardness or softness of biological tissues with respect to an imaging target region in an object to be examined using ultrasonic waves.

Background Art

An ultrasonic diagnostic apparatus is for constructing a tomographic image such as B-mode image by transmitting an ultrasonic wave to the body of an object using an ultrasonic probe and receiving from the body of the object the reflected echo signal of the ultrasonic wave in accordance with the structure of biological tissues, and displaying it for a diagnostic purpose.

Recently, construction of elastic images that present elasticity of biological tissues has been implemented by measuring an ultrasonic reception signal by compressing the object using an ultrasonic probe manually or automatically and obtaining displacement of the respective tissues caused by the compression based on the frame data of two ultrasonic reception signals measured at different times.

Various sorts of elements such as strain or elasticity modulus of biological tissues are known as physical quantities related to elasticity of biological tissues. Here, strain is a relative value obtained by performing spatial differentiation on displacement that is the moving distance of the tissues, and elasticity modulus is a quantitative value wherein the stress change exerted on each region of biological tissues is divided by the strain. Thus it is necessary to measure the pressure to be exerted on the biological tissues in order to obtain the elasticity modulus. An ultrasonic diagnostic apparatus for measuring the pressure exerted on biological tissues by placing a pressure sensor around the transducers of an ultrasonic probe and indirectly measuring the pressure applied on an object is disclosed (for example, Patent Document 1).

Patent Document 1: JP-A-2004-267464

The pressure sensor disclosed in Patent Document 1 is capable of measuring the pressure between the object and the peripheral part of the transmitting/receiving surface of ultrasonic waves, but not capable of measuring the pressure in the ultrasonic waves scanning region immediately beneath the transmitting/receiving surface where ultrasonic waves are to be scanned.

In other words, precisely accurate pressure in the ultrasonic transmission/reception region cannot be obtained by the technique disclosed in the Patent Document 1 since the pressure in the ultrasonic scanning region is merely presumed based on the pressure information in the periphery part of the ultrasonic transmitting/receiving surface. Therefore, there is a possibility that the accuracy of elasticity modulus using the presumed pressure is lowered.

EP 1629 777 A1 describes an ultrasound probe including an ultrasound wave transmit/receive surface coming into contact with a contact surface of a subject, and a pressing mechanism for performing a pressing operation for applying a pressure to the contact surface.

US 2004/210136 A1 describes that elastography is used to examine soft tissue of the uterus to detect tumors and to evaluate the strength of the cervix of the uterus based on its elastographic properties.

US 5 265 612 A describes an intracavity ultrasonic device for elasticity imaging of human tissue by using a probe that has an ultrasonic transducer at one end thereof. An outer elastic sheath surrounds the probe and is sealed so that it can be filled with a fluid.

Given this factor, the object of the present invention is to obtain highly accurate elasticity modulus by performing actual measurement of the pressure exerted on the ultrasonic scanning region.

Disclosure of the Invention

Further, in the object is solved by the present invention according to the independent claims. Further preferred developments are described by the dependent claims.

As mentioned above, in accordance with the present invention, by measuring the pressure added to the ultrasonic scanning region, it is possible to obtain highly accurate elasticity modulus.

Brief Description of the Diagrams

Fig. 1 shows the general configuration of the present invention.

Fig. 2 illustrates compression mechanism of the ultrasonic probe related to the present invention.
Fig. 3 shows a liquid charging/discharging operation unit of the present invention.
Fig. 4 shows a first illustrative example.
Fig. 5 shows the first embodiment of the present invention.
Fig. 6 shows a second embodiment of the present invention.
Fig. 7 shows the second embodiment of the present invention.
Fig. 8 shows the second embodiment of the present invention.
Fig. 9 shows a catheter-type compression sensor of the present invention.
Fig. 10 shows a pattern related to the present invention for measuring pressure from outside of a compression bag.
Fig. 11 shows a pattern related to the present invention for measuring pressure from outside of a compression bag.
Fig. 12 shows a configuration of flow volume sensor related to the present invention.
Fig. 13 shows a configuration of the flow volume sensor related to the present invention.
Fig. 14 shows a configuration of the flow volume sensor related to the present invention.
Fig. 15 shows a configuration of the flow volume sensor related to the present invention.
Fig. 16 shows a configuration of the flow volume sensor related to the present invention.
Fig. 17 shows automatic compression mechanism of the present invention.
Fig. 18 shows a pattern related to the present invention for measuring pressure from outside of a compression bag.

Best Mode for Carrying Out the Invention

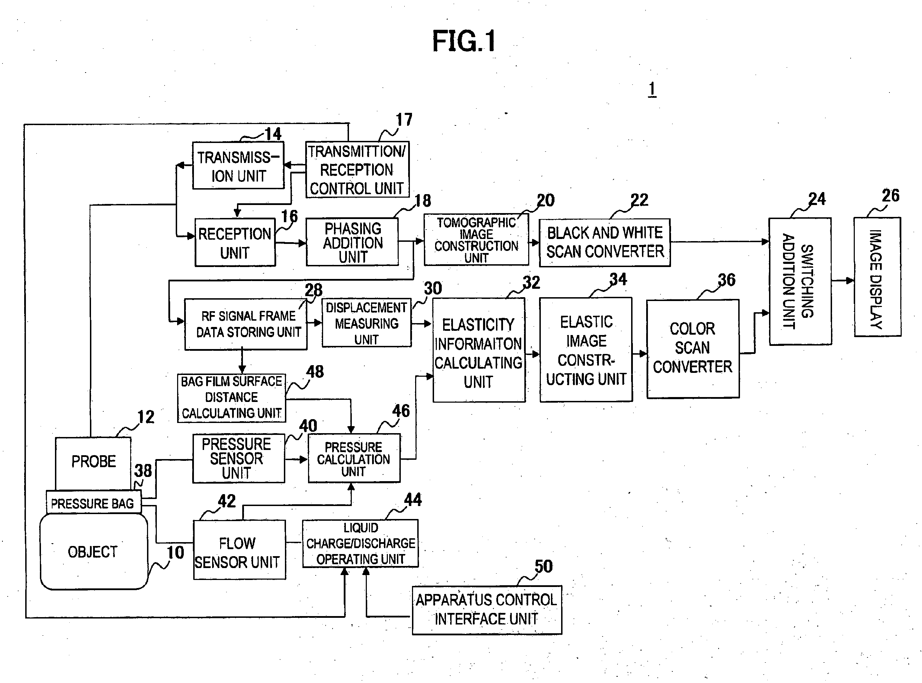

An example of an ultrasonic probe and ultrasonic diagnostic apparatus to which the present invention is applied will be described using a diagram. Fig. 1 is a block diagram showing the configuration of an ultrasonic diagnostic apparatus to which the present invention is applied.

As shown in Fig. 1, an ultrasonic diagnostic apparatus 1 comprises:

an ultrasonic probe 12 for applying to an object 10;
a transmission unit 14 for repeatedly transmitting an ultrasonic wave to the object 10 via the ultrasonic probe 12 at a constant time interval;
a reception unit 16 for receiving time series of reflected echo signals generated from the object 10;
a transmission/reception control unit 17 for controlling the transmission unit 14 and the reception unit 16; and
a phasing addition unit 18 for phasing and adding the reflected echoes received in the reception unit 16.

It also comprises:

a tomographic image constructing unit 20 for constructing a grayscale tomographic image such as a black and white tomographc image of the object based on the RF signal frame data from the phasing addition unit 18; and
a black and white scan converter 22 for converting the output signals of the tomographic image constructing unit 20 into the signals to correspond with a display of the image display device 26.

It also comprises:

an RF signal frame data storing unit 28 for storing the RF signal frame data outputted from the phasing addition unit 18;
a displacement measuring unit 30 for selecting at least two sets of frame data from the RF signal frame data storing unit 28 and measuring displacement of the biological tissues of the object 10;
an elasticity information calculating unit 32 for obtaining strain or elasticity modulus from the displacement information measured in the displacement measuring unit 30;
an elastic image constructing unit 34 for constructing a color elastic image from the strain or elasticity modulus calculated in the elasticity information calculating unit 32; and
a color scan converter 36 for converting the output signals of the elastic image constructing unit 34 into the signals to correspond to the display of the image display device 26.

It also comprises:

a switching/adding unit 24 for superimposing, juxtaposing or switching a black and white tomographic image and a color elastic image; and
the image display device 26 for displaying the combined composite image.

A compression bag is also comprised between the object 10 and the ultrasonic probe 12 for measuring the pressure. The compression bag 38 is made of a material capable of getting through ultrasonic waves, and is placed on the ultrasonic scanning surface of the ultrasonic probe 12. The compression bag 38 is formed by a film made of a material that is safe for a living body such as polyurethane, vinyl chloride, latex (natural rubber) or silicon.

Liquid such as water or oil is filled inside of the compression bag 38. A liquid charging/ discharging operation unit 44 is provided to the compression bag 38 for expanding or deflating the bag by charging/discharging liquid to/from the compression bag 38.

Through the operation of the liquid charging/discharging operation unit 44, compression to the object 10 is increased when the liquid is discharged from the compression bag 38 whereby deflating the bag, and compression to the object 10 is reduced when the liquid is charged to the compression bag 38 whereby extending the bag. While operation of the liquid charging/discharging operation unit 44 is to be executed by a device control inter face unit 50 for automatic control, it also is designed as capable for manual operation.

The ultrasonic diagnostic apparatus 1 also comprises:

a flow sensor unit 42 for measuring the flow volume of the liquid charged/discharged by the liquid charging/discharging operation unit 44;
a pressure sensor 40 for measuring the pressure (water pressure) inside of the compression bag 38;
a compression bag film surface distance calculating unit 48 for measuring the flow volume of the liquid charged/ discharged by the RF signal frame data of the RF signal frame data storing unit 28; and
a pressure calculating unit 46 for calculating the pressure of the ultrasonic scanning region of the object 10 to which the compression bag is applied, from the flow volume information measured in the flow sensor unit 42 or the compression bag film surface distance calculating unit 48 or the pressure information of the pressure sensor 40.

The pressure information calculated in the pressure calculating unit 46 is inputted to the elasticity information calculating unit 32, and elasticity modulus is obtained from displacement information of the displacement measuring unit 30. The details thereof will be described later.

Here, the general configuration of the ultrasonic diagnostic apparatus 1 will be described in detail.

The ultrasonic probe 12 is formed having a plurality of transducers, and a function for transmitting/receiving ultrasonic waves to the object 10 via the transducers. The transmission unit 14 produces transmission pulses for generating ultrasonic waves by driving the ultrasonic probe 12, and has the function for setting a convergent point of the ultrasonic waves to be transmitted. Also, the reception unit 16 is for generating RF signals that are reception signals by amplifying the reflected echo signals received by the ultrasonic probe 12 by a predetermined gain.

The phasing addition unit 18 is for generating RF signal frame data by inputting and phase controlling the RF signals amplified in the reception unit 16 and forming ultrasonic beams with respect to one or a plurality of conversion points.

The tomographic image constructing unit 20 is for obtaining tomographic image data by inputting the RF signal frame data from the phasing addition unit 18 and performing signal processing such as gain compensation, log compression, detection, edge enhancement and filtering process.

Also, the black and white scan converter 22 is configured including an A/D converter for converting the tomographic image data from the tomographic image constructing unit 20 into digital signals, a frame memory for storing the converted plural sets of tomographic image data in chronological order, and a controller.

The black and white scan converter 22 is for obtaining the tomographic frame data of the object stored in the frame memory as one image, and reading out the obtained tomographic frame data by TV synchronism.

The RF frame data storing unit 28 is for storing the plural sets of RF signal frame data from the phasing addition unit 18. The displacement measuring unit 30 selects a pair of (that is two sets of) RF signal frame data from the RF signal frame data group stored in the RF frame data storing unit 28.

For example, it stores the RF signal frame data generated from the phasing addition unit 16 based on the time series that is the frame rate of the image to the RF frame data storing unit 28 in order, selects the stored RF signal frame data (N) as a first set of data, at the same time as selecting one set of RF signal frame data (X) from the RF signal frame data group (N-1, N-2, N-3, -, N-M) stored in times past.

Here, N, M and X are index numbers appended to the RF signal frame data, and to be whole numbers.

Then the displacement measuring unit 30 obtains one-dimensional or two-dimensional displacement distribution related to a movement vector that is the direction and size of the displacement in the biological tissues corresponding to each point of the tomograhic image by performing one-dimensional or two-dimensional correlation processing from the selected pair of data that is RF signal frame data (N) and RF signal frame data (X).

Here, the block matching method is to be used for detecting a movement vector. The block matching method divides an image into, for example, blocks formed by NXN pixels, focuses attention to a block within the region of interest, searches for the block which is most approximated to the focused block from the previous frame and performs the process to determine a sample value by predictive coding that is difference, referring to the searched block.

The elasticity information calculating unit 32 is for calculating strain or elasticity modulus of the biological tissues which correspond to each point of the tomographic image from the measured value outputted from the displacement measuring unit 30 such as a movement vector and the pressure value outputted from the pressure calculating unit 46, and generating elastic image signals that are elastic frame data based on the calculated strain or elasticity modulus.

At this time, the strain data is calculated by performing spatial differentiation on the moving distance or displacement of the biological tissues. Also, data of elasticity modulus is calculated by dividing pressure change by the strain change. For example, when displacement measured by the displacement measuring unit 30 is set as L(X) and the pressure measured by the pressure calculating unit 46 is set as P(X), the strain $\Delta S(X)$ can be calculated by performing spatial differentiation on L(X) thus can be obtained using a formula which is $\Delta S(X) = \Delta L(X)/\Delta X$.

Also, Young's modulus Ym(X) of elasticity modulus data can be calculated by the formula which is: Ym = $(\Delta P(X))/\Delta S(X)$. Since elasticity modulus of the biological tissues equivalent of each point of the tomographic image can be obtained from the previously described Young's modulus, two-dimensional elastic image data can be continuously obtained. Young's modulus is the ratio between simple tension stress added to an object and the strain that is generated parallel to the tension.

The elastic image constructing unit 34 is configured including the frame memory and an imaging processing unit, and is for storing the elasticity frame data outputted from the elasticity information calculating unit 32 in chronological order and performing image processing with respect to the stored frame data.

The color scan converter 36 has a function for appending hue information to the elasticity frame data from the elastic image constructing unit 34, that is for converting the frame data into red (R), green (G) and blue (B) which are the light's three primary colors based on the elastic frame data. For example, it converts the elasticity data having large strain into a red color code at the same time as converting the elasticity data having small strain into a blue color code.

A switching/adding unit 24 related to the present invention comprises the frame memory, the image processing unit and an image selecting unit. Here, the frame memory is for storing the tomogrpahic image data from the black and white scan converter 32 and the elastic image data from the color scan converter 36.

Also, the image processing unit is for synthesizing the tomographic image data stored in the frame memory and the elastic image data by changing the synthesis ratio. Luminance information and hue information of the respective pixels of the synthesized image can be calculated by adding the respective information of the black and white image and the color elastic image by the synthesis ratio. Further, the image selecting unit is for selecting the image to be displayed on the image display device 26 from among the tomographic image data and elastic image data in the frame memory and the synthesized image data in the image processing unit.

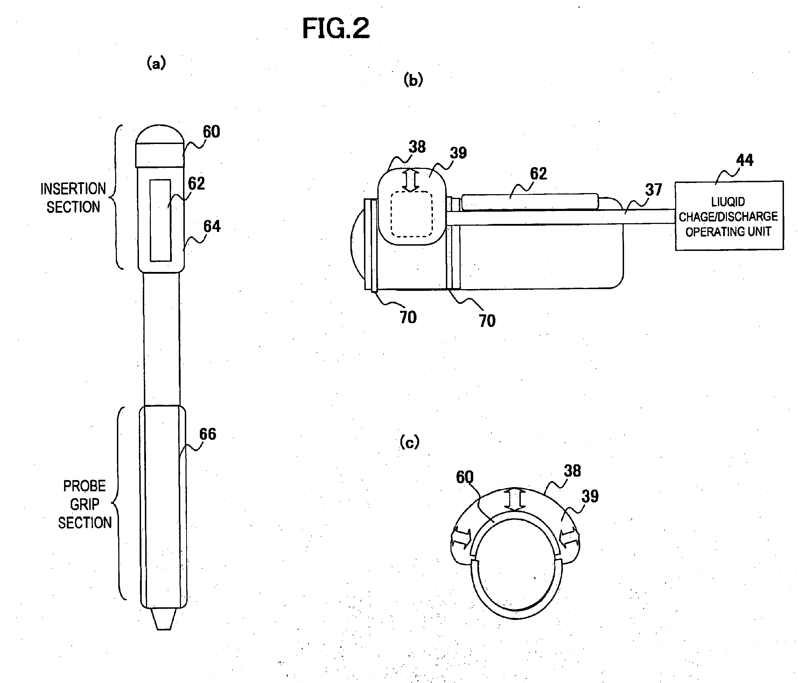

Here, the ultrasonic probe 12 will be described. Fig. 2 shows the lateral view of the ultrasonic probe 12. The ultrasonic probe 12 is a body inserting-type, and has a circular cylinder shape for inserting in the body of the object. The end portion of the ultrasonic probe 12 in the longitudinal direction has spherical shape, and the other end portion is joined to a cable being connected to the transmission unit 14 or the reception unit 16 of the ultrasonic diagnostic apparatus 1.

The vicinity of an end-portion of the ultrasonic probe 12 in longitudinal direction is an insertion unit 64 for being inserted into the body of the object 10, and a plurality of transducers are arranged therein. For example, the insertion unit 64 can be inserted in the region where a prostate gland can be observed, and RF signals of the prostate gland can be obtained by transmitting/receiving the ultrasonic waves using the arranged ultrasonic transducers.

A plurality of ultrasonic transducers are arranged in the insertion unit 64 which are formed as convex-type probe 60 in the front portion and as linear-type probe 62 in the posterior portion, and the respective ultrasonic transducers are connected to the transmission unit 14 or the reception unit 16 via a cable.

Meanwhile, a grip portion 65 for an operator to hold the ultrasonic probe 12 is provided on the end of the probe that is connected to the cable. The operator can hold the grip portion 65 and arbitrarily move the ultrasonic probe 12.

The configuration wherein the compression bag 38 is placed on the convex-type probe 60 of the ultrasonic probe 12 shown in Fig. 2(a) is illustrated in Fig. 2(b) and Fig. 2(c). Fig. 2(b) shows the lateral view of the ultrasonic probe 12 in the major-axis direction, and Fig. 2(c) is the lateral view of the ultrasonic probe 12 in the minor-axis direction.

The compression bag 38 is placed so as to cover the periphery of the convex-type probe 60, and both ends of the compression bag 38 are fixed by two fixing belts 70. A hollow tube 37 is placed along the ultrasonic probe 12 toward the major-axis direction, and is for coupling the compression bag 38 and the liquid charging/discharging operation unit 44.

By injecting liquid into the tube 37 in the liquid charging/discharging operation unit 44, the liquid is charged to the compression bag 38 whereby expanding the compression bag 38. Also, by extracting liquid from the tube 37 in the liquid charging/discharging operation unit 44, the liquid is extracted from the compression bag 38 to the tube 37, whereby deflating the compression bag 38.

Concretely, when liquid is injected into the compression bag 38, the compression bag 38 is expanded in radial pattern centering around the center portion of the minor-axis cross-section of the ultrasonic probe 12.

The compression bag 38 may be configured having a ring-shape that covers the whole periphery surface of the ultrasonic probe 12 not only the surface of the convex-type probe 60. In that case also, the compression bag 38 is expanded in a radial pattern centering around the center portion of the minor-axis cross-section of the ultrasonic probe 12.

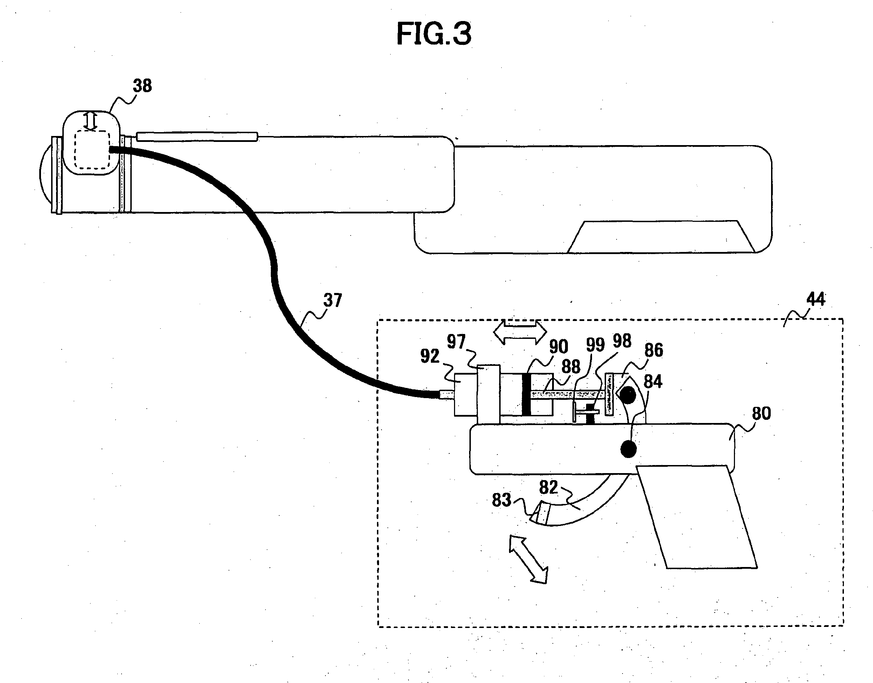

Next, the liquid charging/discharging operation unit 44 will be described using Fig. 3. The liquid charging/ discharging operation unit 44 is mainly formed by a main body unit 80, a cylinder 92 which is fixed to the main body unit 80 and liquid is filled therein, a spin 90 and a pusher 88 which are placed in the cylinder 92 for pushing out or pulling out liquid, a pusher fixing portion 86 for fixing the pusher 88, an operating unit 82 for activating the pusher 88 and a stroke adjusting unit 99 for restricting the movement strokes of the piston 90.

The operating unit 82 passes through the axis of the main body 80 by the supporting unit 84 and is connected to the main body. The operating unit 82 can be rotated centering around the supporting unit 84. One end of the operating unit 82 is coupled to the pusher fixing unit 86, and the other end of the operating unit 82 is a grip 83 for an operator to hold and exert the movement.

Also, one end of the pusher 88 is couple to the pusher fixing portion 86. The other end of the pusher 88 is a piston 90 in the cylinder 92. By reciprocating the piston 90 in the cylinder 92, external force is provided to the liquid inside of the cylinder 92. The liquid to which the external force is applied being pushed by the pusher 88 reaches to the compression bag 38 via the tube 38, and expands the compression bag 38 for the amount of the liquid being pushed out. Contrarily, by pulling out the pusher 88 the liquid in the compression bag 38 is pulled out to the cylinder 92 and deflates the compression bag 38.

The operator can push out the pusher 88 by supporting his/her palm with the main body unit 80 and pull the grip 83 in the lower right direction by grasping with a plurality of fingers, and expand the compression bag 38 according to the amount of the liquid pushed out by the motion of the pusher 88. By grabbing the grip and pushing it in the upper left direction, the pusher 88 can be pulled out, whereby expanding the compression bag 38 for the amount of the liquid pulled out by the motion of the pusher 88.

The stroke adjusting unit 99 is placed in a holding portion 98 which is placed on the stroke surface of the pusher 88 of the main body unit 80. The stroke adjusting unit 99 is a male screw, and has a female screw for the male screw to pass through. By rotating the stroke adjusting unit 99, the stroke adjusting unit 99 can be moved to right and left via the holding portion 98.

Next, function of the stroke adjusting unit 99 will be described. When the piston 90 is exercised to the left direction of the diagram, the pusher 88 contacts the stroke adjusting unit 99 at a predetermined position. Even when an attempt is made to move the piston 90 to the left direction from the position thereof, the piston 90 cannot be exercised to the left direction from the contacted position since the pusher 88 is fixed by the stroke adjusting unit 99. Thus the stroke adjusting unit 99 can restrict the movement stroke of the piston 90.

In this way, the stroke adjusting unit 99 can arbitrarily set the flow volume of the liquid to be injected into the compression bag 38 by restricting movement of the piston 90. In concrete terms, if the surface area of the compression bag 38 is 1000mm$^2$, the preferable injection volume of the liquid per one stroke is in the range of about 0.2cc - 1.0cc.

Because compressing the object 10 too much will cause some problems in obtaining preferable strain or elasticity modulus. Given this factor, in order to make the multiplication of the cross-sectional area of the cylinder 92 times moving distance of the piston 90 to be 0.2cc - 1.0cc, the stroke adjusting unit 99 adjusts the moving distance of the piston 90. Concretely, when the cross-sectional area of the cylinder 92 is set as "S" and the moving distance of the pusher 88 is set as "A", adjustment is to be made so that S×A turns out to be in the range of 0.2cc - 1.0cc.

Also, the syringe unit formed by the cylinder 92, the piston 90 and the pusher 88 has configuration capable of being attached/detached to/from the main body unit 80 at one's fingertips via the holding portion 97. The holding portion 97 is for receiving the cylinder 92 and fitting it together by insertion, and it is fixed to the main body 80 by the holding unit 97 receiving the cylinder 92. Also, the main body unit 80 is configured of a rust-resisting material such as aluminum, stainless or plastic.

While manual compression by an operator is described here, it may be executed by comprising a motor in the liquid charging/discharging operation unit 44 to reciprocate the pusher 88 by the motor for expanding/deflating the compression bag 38.

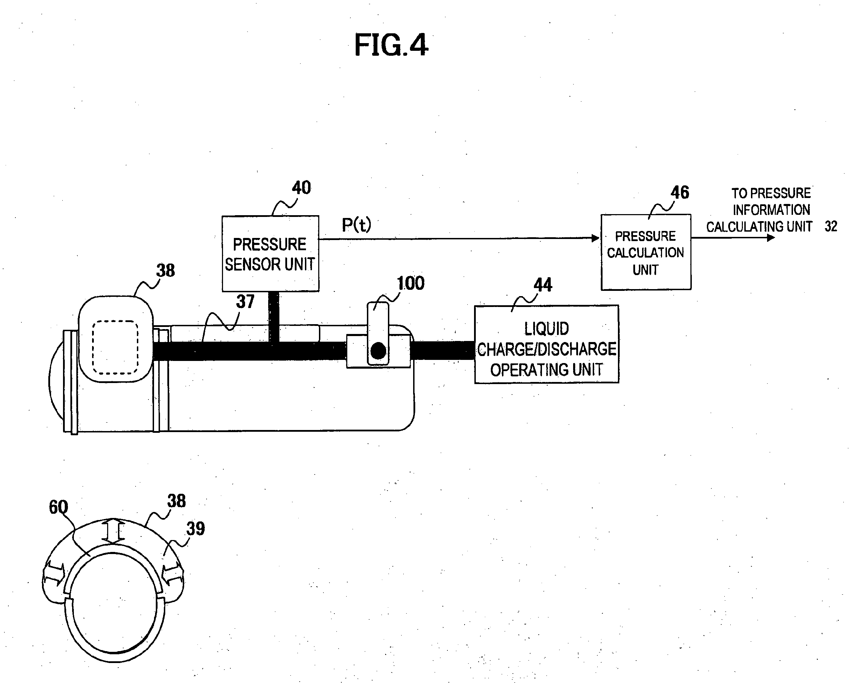

Here, the first example for obtaining the pressure in the ultrasonic scanning region of the object 10 to which the compression bag is applied will be described using Fig. 1 - Fig. 5.

The compression bag 38 is placed so as to cover the convex-type probe 60 that is the ultrasonic probe 12. The compression bag 38 is coupled to the liquid charging/ discharging operation unit 44 and the pressure sensor unit 40 via the tube 37. Pressure information "P" detected by the pressure sensor unit 40 for measuring the pressure in the compression bag 38 is to be outputted to the pressure calculating unit 46. The pressure calculating unit 46 is for calculating the pressure of the ultrasonic scanning region of the object 10 to which the compression bag 38 is applied, from the pressure information of the pressure sensor 40. The pressure information calculated in the pressure calculating unit 46 is inputted to the elasticity information calculating unit 32 as shown in Fig. 1.

A cock 100 for controlling charging/discharging of liquid is placed between the compression bag 38 and the liquid charging/discharging operation unit 44. When the cock 100 is opened, liquid is charged/discharged freely between the compression bag 38 and the liquid charging/discharging operation unit 44. When the cock 100 is closed, the volume of liquid becomes constant since the liquid cannot be charged/discharged between the compression bag 38 and the liquid charging/discharging operation unit 44.

First, in the condition that the compression bag 38 is not applied to the object 10, the pressure sensor 40 measures the pressure in the compression bag 38 and stores the pressure value in a memory of the pressure calculating unit 46 temporarily. Then the pressure sensor 40 measures the pressure in the compression bag 38 in the condition that the compression bag 38 is applied on the object 10 and the object 10 is being compressed. At this time, the volume of liquid in the compression bag 38 is constant before and after the compression. The pressure calculating unit 46 calculates the difference between the pressure before compression which is stored in the memory and the pressure after compression, and outputs the difference of the pressures to the elasticity information calculating unit 32 as the pressure of the ultrasonic scanning region of the object 10 to which the compression bag 38 is applied.

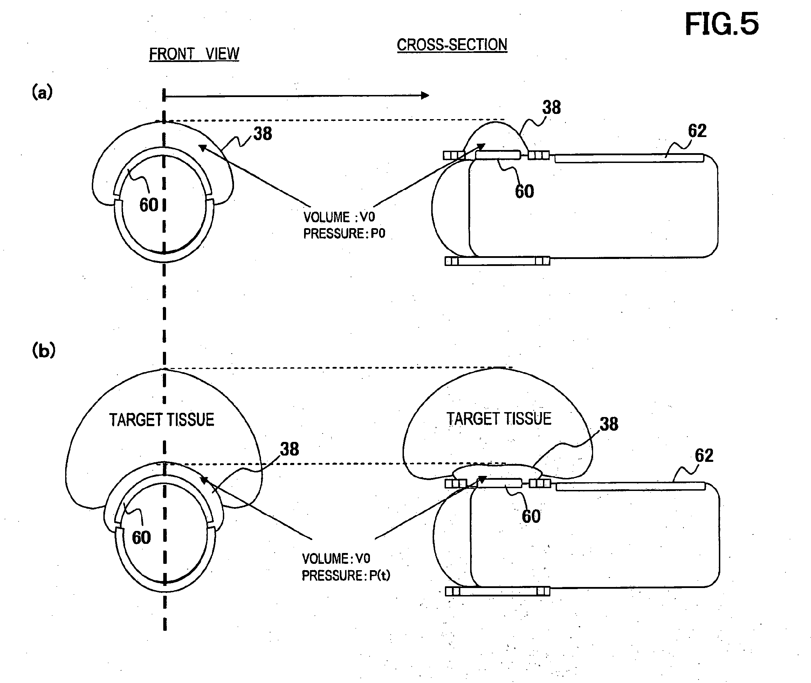

Next, the first embodiment will be described in concrete form using Fig. 5. Fig. 5(a) shows a condition wherein the compression bag 38 is not applied on a target tissue of the object 10. First, in such condition, the pressure calculating unit 46 obtains the relationship between the pressure and the volume inside of the compression bag 38. Then the liquid of volume "V0" is injected to the compression bag 38 from the liquid charging/discharging operation unit 44, and the cock 100 is closed. By closing the cock 100, the volume of the liquid in the compression bag 38 constantly stays as a steady value "V0". In such condition, the pressure "P0" is obtained. This condition is referred to as the reference condition "P0" and the operation thereof is referred to as "calibration".

Fig. 5(b) is a condition wherein the compression bag 38 is applied to the target tissue of the object 10 that is a condition to apply for elasticity diagnosis of the tissue. The ultrasonic probe in such condition is applied to biological tissues of the object, for example, a prostate gland while an operator compresses the ultrasonic probe 12 onto the prostate gland, and the pressure calculating unit 46 obtains pressure information P(t) at an arbitrary time "t" during the compression process of the target tissue. At this time, difference from the reference condition P(t) is the pressure Ptarget(t) being added to the biological tissues of the object 10 at the present time "t", and can be obtained in the pressure calculating unit 46 using the following formula 1:

$$\{Formula\ 1\}\quad Ptarget(t) = P(t) - P0.$$

When the time wherein the RF signal frame before compression is obtained is set as t-1 and the time wherein the RF signal frame data after compression is obtained is set as the present time "t", at the present time "t", the change of compression pressure $\Delta P(t)$ of the biological tissue can be obtained in the pressure calculating unit 46 using the following formula 2:

$$\{Formula\ 2\}\quad \Delta P(t) = Ptarget(t) - Ptarget(t-1).$$

Based on the measurement value on the basis of displacement of the RF signal frame data obtained at the time of "t-1" and "t" in the displacement measuring unit 30 and information of $\Delta P(t)$ obtained in the pressure calculating unit 46, elasticity modulus is calculated in the elasticity information calculating unit 32.

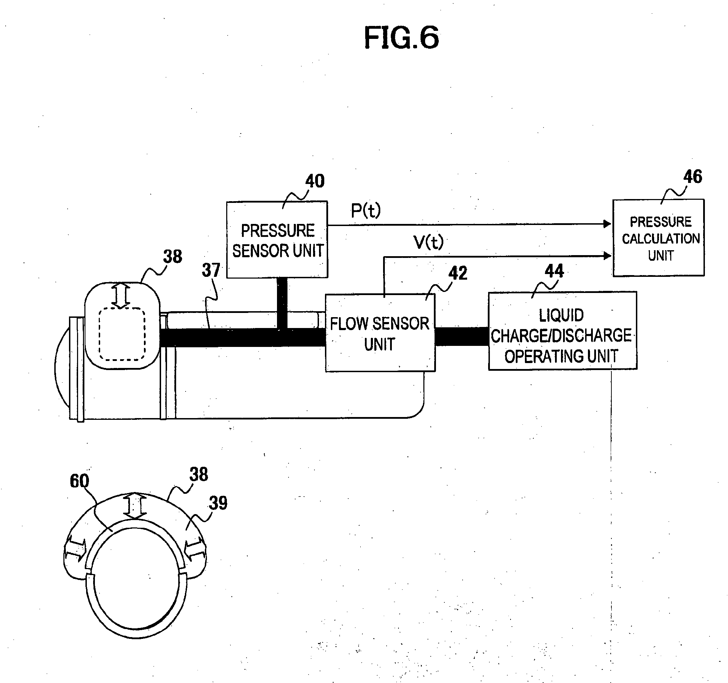

Next, a second embodiment for obtaining pressure of the ultrasonic scanning region of the object 10 to which the compression bag 38 is applied will be described using Fig. 6 - Fig. 8. The difference from the first embodiment is that the flow sensor unit 42 is comprised for detecting inflow/outflow volume of liquid, and pressure of the ultrasonic scanning region of the object 10 to which the compression bag 38 is applied is obtained from the pressure information detected in the pressure sensor unit 40 and the flow volume information detected in the flow sensor unit.

It is configured so that the pressure sensor unit 40 for measuring the pressure in the compression bag 38 is coupled to the compression bag 38, and the pressure information "P" detected in the pressure sensor unit 40 is to be outputted to the pressure calculating unit 46. The flow sensor unit 42 is placed between the compression bag 38 and the liquid charging/discharging operation unit 44, and the flow (volume) information "V" of the liquid charged/discharged to/from the compression bag 38 is to be outputted to the pressure calculating unit 46. The flow sensor 42 has a moving member therein such as a valve or fan that moves along with the movement of liquid, for measuring the flow volume of liquid by the displacement of the moving member.

First, liquid is charged/discharged to/from the compression bag 38 using the liquid charging/discharging operation unit 44 in the condition that the compression bag 38 is not applied to the object 10, the pressure in the compression bag 38 is measured by the pressure sensor unit 40, and flow volume of the charged/discharged liquid is measured by the flow sensor unit 42. Relationship between the measured pressure and the flow volume is temporarily stored respectively in the memory in the pressure calculating unit 46.

Then in a condition that the compression bag 38 is applied to the object 10 for adding compression, liquid is charged/ discharged to/from the compression bag 38 using the liquid charging/discharging operation unit 44, the pressure in the compression bag 38 is measured by the pressure measuring unit 40, and the flow volume of the charged/discharged liquid is measured in the flow sensor unit 42. The pressure calculating unit 46 calculates difference of the pressure at a predetermined flow volume value based on the relationship between the respective pressures and flow volumes measured at this time and the previously mentioned respective pressures and flow volumes that are temporarily stored in the memory, and the calculated pressure difference is outputted to the elasticity information calculating unit 32 as the pressure of the ultrasonic scanning region of the object 10 to which the compression bag 38 is applied.

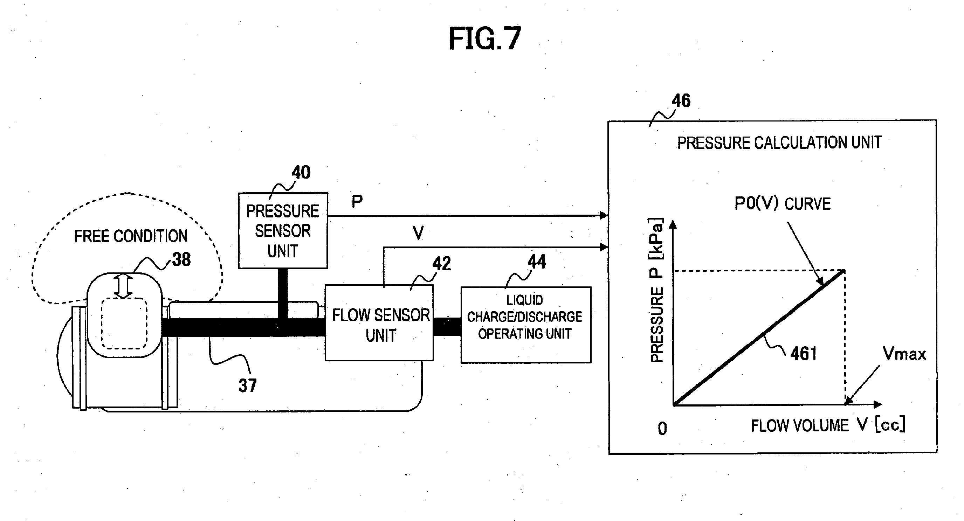

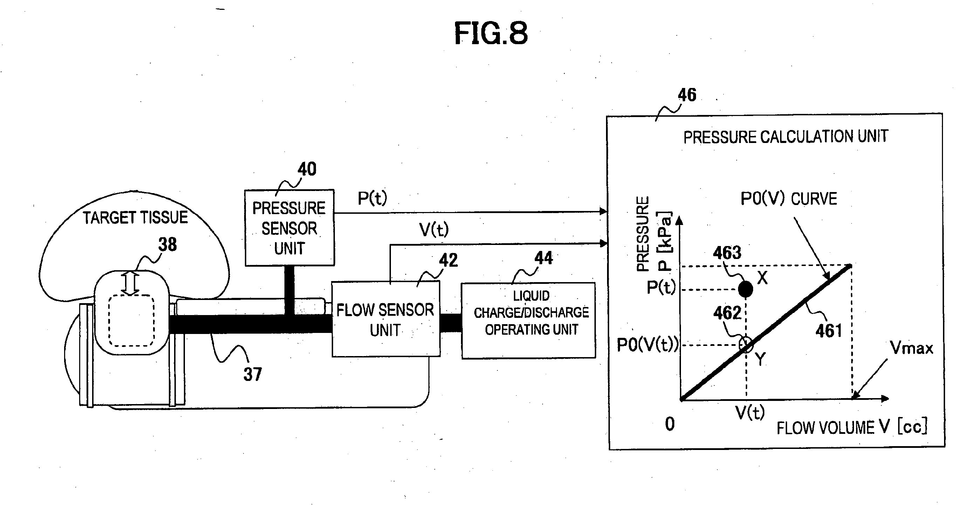

Next, the second embodiment will be described in concrete term using Fig. 7 and Fig. 8. Fig. 7 shows the condition that the compression bag 38 is not applied to the target tissue of the object 10, and the pressure calculating unit 46 obtains the relationship between pressure and volume in the compression bag 38. This relationship is obtained in a volume range of the flow [0,Vmax] (Vmax is around several cc) that is set in advance to suit the tissue compression. A solid line 461 of the graph in Fig. 7 shows relationship of the pressure with the flow volume when the range of the flow volume obtained in the pressure calculating unit 46 is 0 - Vmax. It is obvious from the diagram that the pressure increases as the flow volume increases.

Then diagnosis of tissue elasticity is carried out, and the target tissue of the object 10 is compressed. The pressure information P(t) and flow (volume) information V(t) are obtained at an arbitrary time "t" during the compression process. The flow (volume) information V(t) is not the flow v(t) passed through a flowmeter at time "t", but the flow volume of the entire liquid volume $V(t) = \Sigma v(t)$ which has charged into the compression bag 38.

In the graph of Fig. 8, the relationship between the flow volume and the pressure obtained in the condition that the target tissue is being compressed is corresponded to the solid line 461 of the graph showing the relationship between the flow and the pressure when the compression bag is not applied to the target tissue.

In the volume V(t) in the reference condition wherein the compression bag 38 is not applied to the target tissue, the amount of internal pressure can be obtained as PO(V(t)). In the graph, (V(t), PO(V(t))) is displayed as a point Y462. Then the pressure obtained in the volume V(t) under the condition wherein the target region is being compressed is P(t). In the graph, (V(t), P(t)) is displayed as a point X463.

The difference from P(t) of the reference condition is the pressure Ptarget(t) of the compression added to the biological tissue at the present time "t", and can be obtained as shown in formula 3 below.

$$\{Formula\ 3\}\quad Ptarget(t) = P(t) - PO(V(t))$$

Here, it may be set so that pressure information and flow (volume) information are to be previously A/D converted in the pressure sensor unit 40 and the flow sensor unit 42, and inputted to the pressure calculating unit 46 as digital signals, and that the pressure information and the flow (volume) information of analogue signals may be A/D converted in the pressure calculating unit 46.

Also, while the example for outputting flow volume information from the flow sensor unit 42 is described above, it may be set so that the flow volume v(t) passed through the flowmeter at the present time is to be outputted so as to evaluate the flow $V(t) = \Sigma v(t)$.

Also, the pressure may be obtained by the pressure calculating unit 46 based on the flow volume pushed back by measuring a small amount of the liquid pushed back to the side of the liquid charging/discharging operation unit 44 due compression added to the target tissue of the object 10. In concrete terms, a plurality of pressure information calculated from a predetermined injection volume of the fluid and the flow volume of liquid pushed back by compression of the compression bag 38 are to be recorded in advance in the pressure calculating unit 46. In the case that the compression bag 38 is expanded and compressed the target tissue of the object 10, the pressure calculating unit 46 obtains the pressure from a predetermined injection volume of liquid and the flow volume of the liquid that being pushed back.

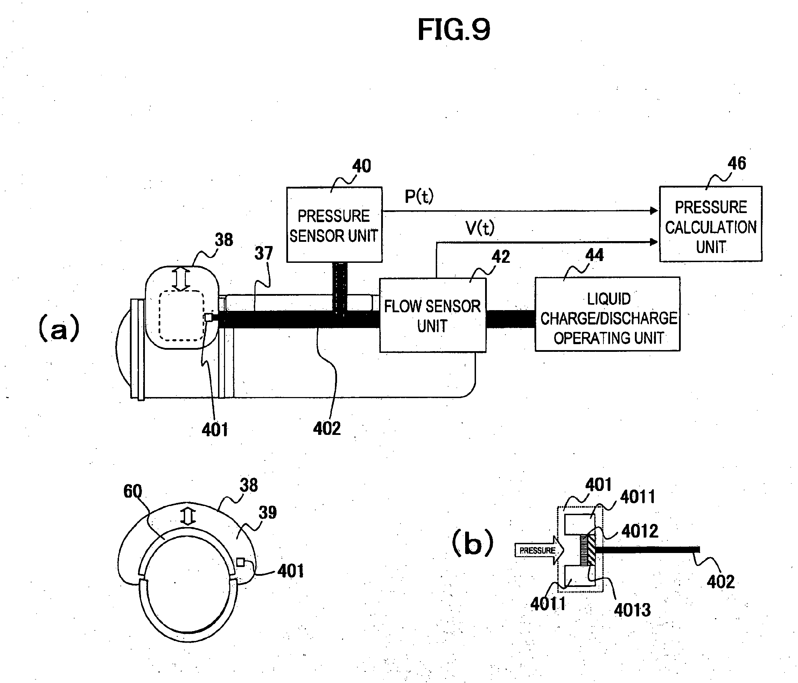

A variety of patterns of the pressure sensor unit 40 to be applied to the above described embodiment 1 and embodiment 2 will be described using Fig. 9 - Fig. 11.

Fig. 9(a) shows the pattern to which the pressure sensor catheter is applied for measuring the pressure in the compression bag 38. A pressure sensor 401 for measuring the pressure in the compression bag 38 is a catheter-type, and is connected to the pressure sensor unit 40 via a cable 402 in a tube 37. The pressure information measured at the end portion of the pressure sensor 401 is transmitted to the pressure sensor unit 40.

Fig. 9(b) shows the details of the pressure sensor 401. The catheter-type pressure sensor 401 has, for example, a hollow body 4011, a diaphragm film 4012 to which the micromachining technique is applied and a strain gage 4013. The diaphragm film 4012 and the strain gage 4013 are placed in the inner periphery surface of the hollow body 4011, and the liquid in the compression bag 38 touches the diaphragm film 4012.

The diaphragm film 4012 is concaved based on the pressure of the compression bag 38. Concave information of the diaphragm film 4012 is detected by the strain gage 4013. Then the concave information detected by the strain gage 4013 is outputted to the pressure sensor unit 40 via the cable 402. In the pressure sensor unit 40, the relationship between the concave information of the diaphragm film and the pressure is measured in advance.

The pressure sensor unit 40 calculates the pressure information based on the measured concave information of the diaphragm of the pressure sensor 401, and outputs it to the pressure calculating unit 46.

Also, the cable 402 itself may be applied to the pressure sensor. From the end-portion to the central portion of the cable 402 may be applied with a material that gets depressed in accordance with the pressure in the compression bag 38 or the tube such as rubber material, and the pressure may be measured based on the concave level. In concrete terms, liquid such as oil or saline is filled inside of the pressure sensor 401. The pressure sensor unit 40 recognizes the quantity of liquid being pushed out in accordance with concave level of the pressure sensor 401 caused by pressure in the compression bag 38 or the tube 37. The pressure sensor unit 40 calculates the pressure information based on the concave information of the pressure sensor 401 and outputs it to the pressure calculating unit 46.

While the pressure sensor 401 is placed in the compression bag 38 here, it may be placed on the surface of the cylinder 92 or inside of the tube 37.

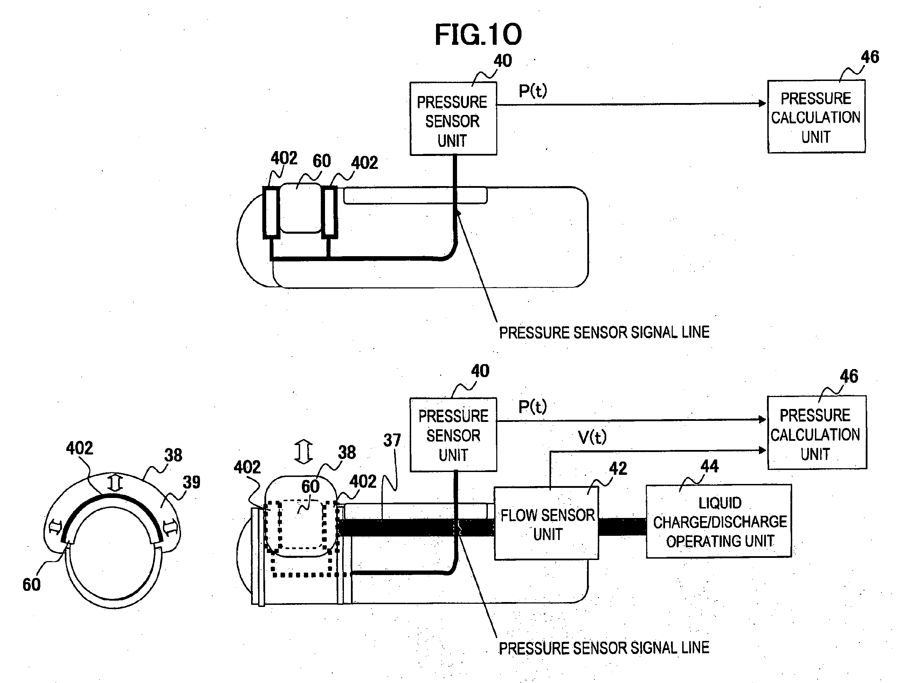

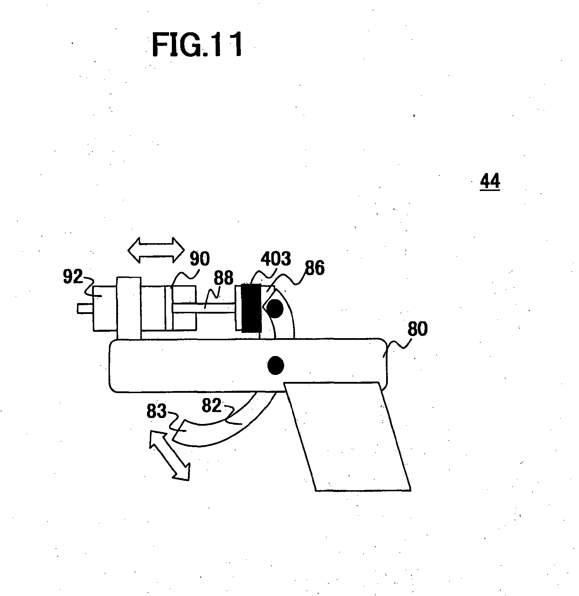

While a pattern for measuring the pressure in the compression bag 38 in the pressure sensor unit 40 is illustrated above, the pattern for measuring the pressure outside of the compression bag 38 is illustrated in Fig. 10 and Fig. 11.

In this pattern, as shown in Fig. 10, the pressure sensor 402 for measuring the pressure in the compression bag 38 is placed between the compression bag 38 and the ultrasonic probe 12. The pressure sensor 402 is made of a non pressure-sensitive material, piezoelectric material such as lead zirconate titanate, or semiconducting pressure sensor, etc. The compression condition of the pressure sensor 402 placed between the compression bag 38 and the ultrasonic probe 12 varies in accordance with expansion or deflation of the compression bag 38. The pressure sensor unit 402 detects the pressure, and the detected pressure information is transmitted to the pressure sensor unit 40. The pressure sensor unit 40 calculates the pressure information of the pressure sensor 402 and outputs it to the pressure calculating unit 46.

Fig. 10 shows the arrangement of the pressure sensor 402 on the periphery of the convex-type probe 60.

When the compression bag 38 is expanded, it produces the condition that the compression bag 38 and the pressure sensor 402 are firmly attached to each other whereby increasing the constriction and pressure of the pressure sensor 402. Contrarily, when the compression bag 38 is deflated, the compression bag 38 is freed with respect to the pressure sensor 402 whereby reducing the pressure caused by constriction of the pressure sensor 402. In this way, it is possible to evaluate the pressure P(t) inside of the compression bag 38 indirectly by measuring the constrictive pressure of the compression bag 38. The above-described process can be carried out even if the pressure sensor is not waterproof, since the pressure sensor 402 does not need to be soaked in liquid.

Also, as shown in Fig. 11, a pressure sensor 403 may be placed on the back of the pusher 88 of the liquid charging/discharging operation unit 44. Stated another way, the pressure sensor 403 is placed between the pusher 88 and the pusher fixing portion 86.

In the condition that the pressure sensor 403 is intervened between the pusher 88 and the pusher fixing portion 86, the piston 90 is reciprocated inside of the cylinder 92, and external force is added to liquid inside of the cylinder 92. The liquid added with external force reaches the compression bag 38 via the tube 37, and expands the compression bag 38 for the portion of the liquid pushed out by motion of the pusher 88 and the piston 90. Contrarily, when the pusher 88 is pulled, liquid in the compression bag 38 is pulled out to the cylinder 92 whereby deflating the compression bag 38. In this motion, the more the compression bag is expanded, the more the pressure in the compression bag 38 and the force to be transmitted to the pusher 88 are increased.

Since the pressure sensor 403 is compressed by the transmission of the pressure from the compression bag 38 to the pusher 88 in compliance with expansion/deflation of the compression bag 38, it is possible to indirectly measure the pressure P(t) loaded on the compression bag 38. In other words, the pressure Ptarget(t) of the compression added to the biological tissue can be obtained using the above-described formula.

The pressure sensor 403 detects the pressure thereof, and the detected pressure information is transmitted to the pressure sensor 40 though not shown in the diagram. The pressure sensor 40 calculates the pressure information of the pressure sensor 403, and outputs it to the pressure calculating unit 46. The above-described process may be carried out even if the pressure sensor is not waterproof since the pressure sensor does not need to be soaked in liquid.

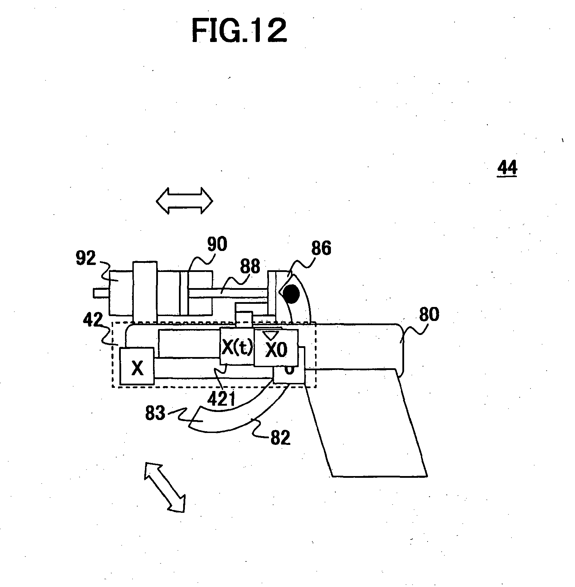

A variety of patterns of the flow sensor unit 42 for applying to the above-described embodiments will be described using Fig. 12 - Fig. 16.

In the pattern shown in Fig. 12, a position sensor 421 in the flow sensor unit 42 is coupled to the pusher fixing portion 86. The position sensor 421 is formed by a general encoder and an optical sensor such as infrared radiation, etc. It is set so that the position of the pusher 88 (= position of the piston 90) is to be detected by the position sensor 421.

Multiplication of the cross-sectional area of the cylinder 92 and the moving distance of the pusher 88 detected by the position sensor 421 is equivalent of the flow volume to be pushed out to the compression bag 38. In other words, flow volume of the liquid charged into the compression bag 38 can be measured by detecting the position of the pusher 88.

For example, when the cross-sectional area of the cylinder 92 is set as "S", the initial position of the pusher 88 is set as "X0" and the position of the pusher is X(Xo≦X ≦Xmax), the flow volume of liquid charged into the compression bag 38 when the position of the pusher is X=X(t) at time "t" can be expressed in the following formula 4:

$$\{\text{Formula 4}\} \quad V(t) = S \times (X(t) \, X0).$$

In accordance with this method, it is possible to measure flow volume of the liquid charged into the compression bag 38 more directly and accurately by using the position sensor 421. The flow sensor unit 42 measures flow volume of the liquid charged/discharged to/from the compression bag 38, and outputs the measured volume information to the pressure calculating unit 46.

In this way, the flow sensor unit 42 can also be placed in the liquid charging/discharging operation unit 44. When it is combined with the pattern shown in Fig. 11, the pressure sensor and the flow sensor can be comprised in the liquid charging/discharging operation unit 44. Therefore, all of the additional devices necessary for the above-described embodiments can be placed in the liquid charging/discharging operation unit 44.

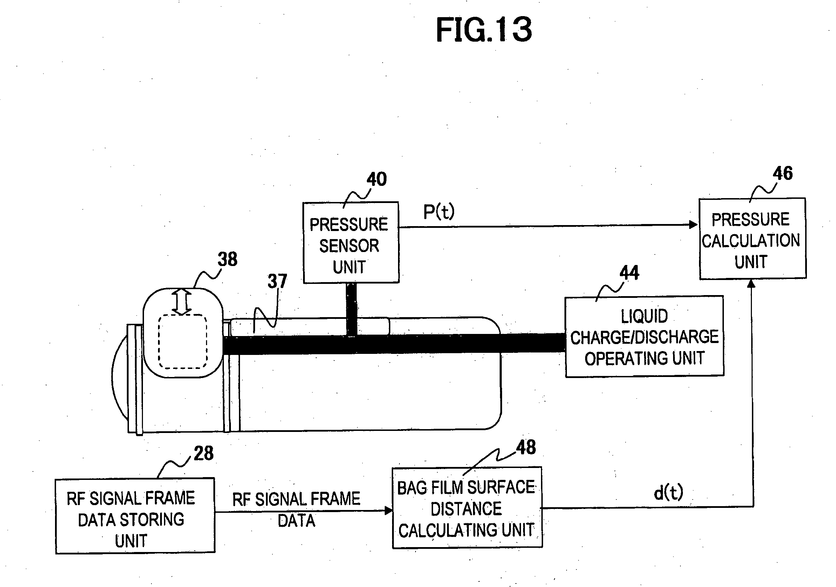

While the pattern to measure the pressure based on the volume information of the liquid charged into the compression bag 38 is described above, the pattern for measuring the pressure added to the target tissue based on the flow volume read out from ultrasonic reception signals will now be described. The pattern for evaluating the expansion of the compression bag 38 by ultrasonic reception signals and measuring the flow volume of the liquid charged/discharged by the liquid charging/ discharging operation unit 44 will be described referring to Fig. 13 - Fig. 16.

As shown in Fig. 13, the present pattern has a compression bag film surface distance calculating unit 48 for calculating the degree of expansion of the compression bag 38 by recognizing the film surface of the compression bag 38 from RF signal from data as shown in Fig. 13. The RF signal frame data storing unit 28 for storing a plurality of RF signal frame data wherein the obtained ultrasonic reception signals are phased and added outputs the RF signal frame data to the compression bag film surface distance calculating unit 48. In the compression bag film surface distance calculating unit 48, distance (compression bag film surface distance) "d" from the transmitting/receiving surface of the ultrasonic probe 12 to the film surface of the compression bag 38 is analyzed using the RF signal frame data, and the result thereof is to be outputted to the pressure calculating unit 46. Also, the pressure sensor unit 40 for measuring the pressure in the compression bag 38 is coupled to the compression bag 38, and the pressure information "P" detected by the pressure sensor unit 40 is to be outputted to the pressure calculating unit 46.

The pressure calculating unit 46 obtains the pressure of the ultrasonic scanning region of the object 10 based on the pressure information obtained from the pressure sensor unit 40 and the film surface distance of the compression bag 38 calculated in the compression bag film surface distance calculating unit 48.

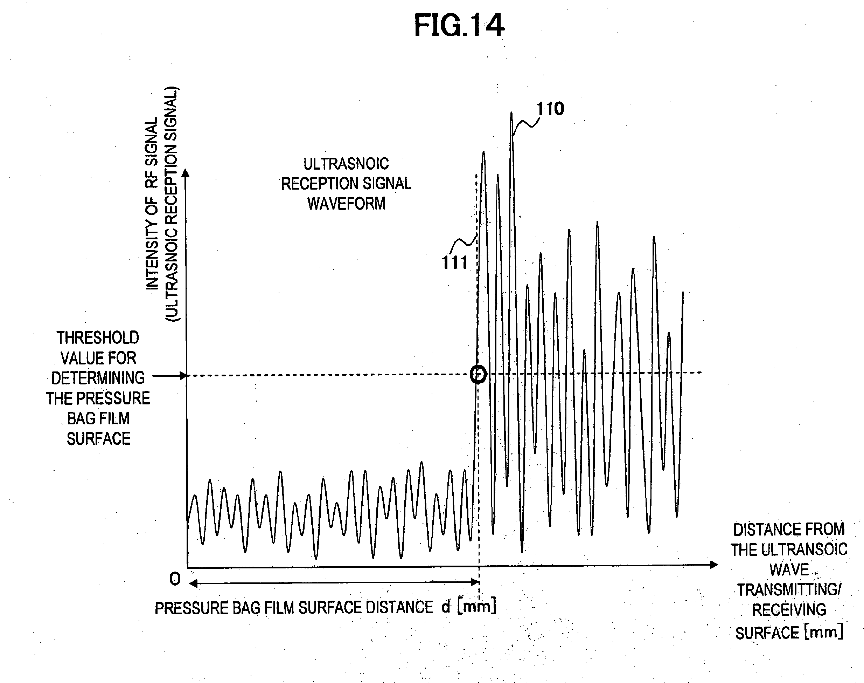

A concrete example of the above-mentioned pattern will be described using Fig. 14. Fig. 14 shows the calculation method for calculating the compression bag film surface distance in the compression bag film surface distance calculating unit 48.

A waveform 110 of the graph is the ultrasonic reception signal waveform being received in one ultrasonic transducer when an ultrasonic wave is transmitted/received by applying the ultrasonic probe to a biological tissue via the compression bag 38. The vertical axis indicates intensity of the RF signal (ultrasonic reception signal), and the lateral axis indicates the distance from the ultrasonic transmitting/receiving surface of the ultrasonic probe 12.

Liquid such as water is filled in the compression bag 38, and water has very weak ultrasonic reflection intensity compared to biological tissues since ultrasonic wave scatterer is not included in water. Also, while the compression bag is made of a very thin film, since it is different from the acoustic impedance of water, the ultrasonic waves produce large reflection at the border between the water and the film surface of the compression bag 38.

By using the compression film surface distance calculating unit 48 for causing an RF signal (ultrasonic reception signal) to set a proper threshold for determining the compression bag film surface as shown in the diagram and obtaining the distance at the time of surpassing the set threshold for the first time by searching from the ultrasonic transmitting/receiving surface, a border position 111 between water and the compression bag can be easily detected. The distance up to this border is obtained as the compression bag film surface distance "d".

First, liquid is charged/discharged to/from the compression bag 38 using the liquid charging/discharging operation unit 44 in the condition that the compression bag 38 is not applied to the compression bag 38, the pressure sensor unit 40 measures the pressure in the compression bag 38, and the compression bag film surface distance calculating unit 48 measures the compression bag film surface distance. The relationship between the respective pressure and the compression bag film surface distance measured at this time is stored in the memory of the pressure calculating unit 46 temporally.

Then in the condition wherein the compression bag 38 is applied for compressing the object 10, the liquid charging/ discharging operation unit 46 charges/discharges liquid to/from the compression bag 38, the pressure sensor unit 40 measures the pressure in the compression bag 38, and the compression bag film surface distance calculating unit 48 measures the compression bag film surface distance. The pressure calculating unit 46 calculates the difference of pressure in a predetermined compression bag film surface distance from relationship of the pressure and the compression bag film surface distance measured respectively at this time and relationship between the pressure which is temporally stored in the memory and the compression bag film surface distance, and outputs the calculated pressure difference to the elasticity information calculating unit 32 as the pressure of the ultrasonic scanning region of the object 10 to which the compression bag is applied.

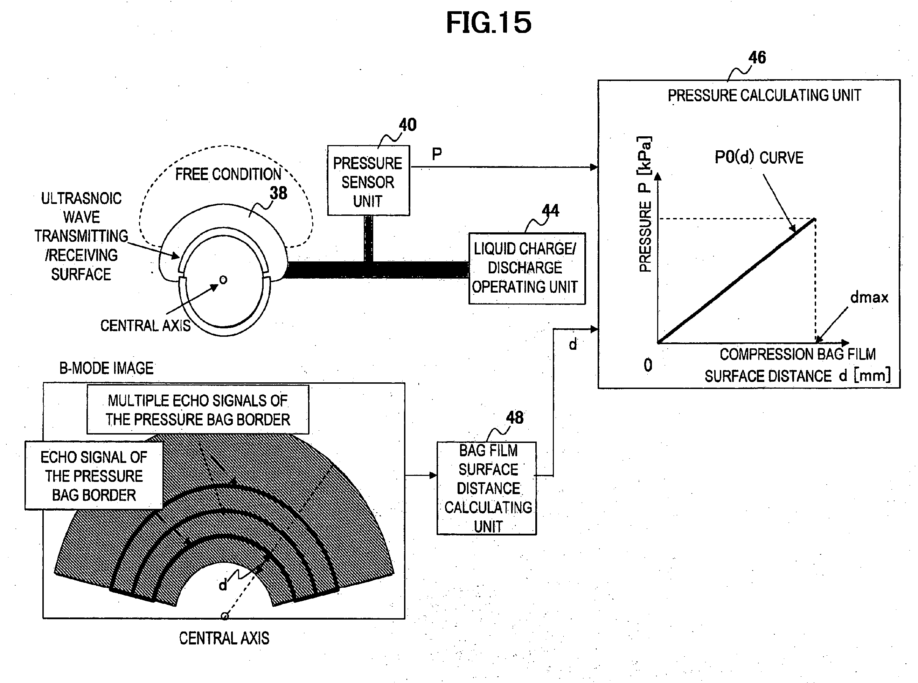

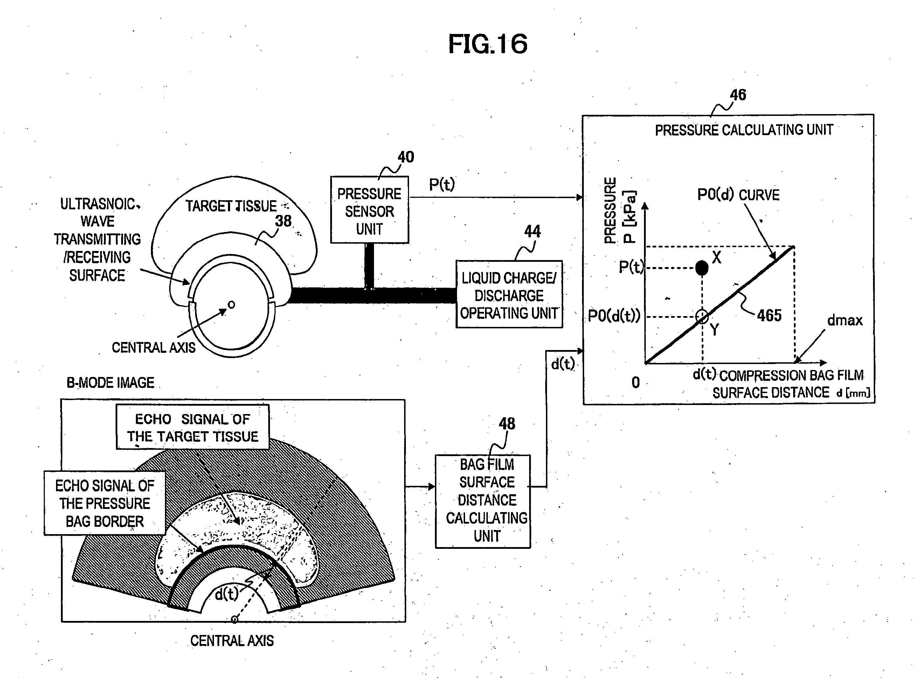

Next, the pattern for measuring the pressure wherein the compression bag 38 is compressing the biological tissue using the pressure information "P" and the compression bag film surface distance information "d" inputted to the pressure calculating unit 46 will be concretely described using Fig. 15 and Fig. 16.

First, relationship between the pressure inside of the compression bag 38 and the compression bag film surface distance is obtained in the condition that the compression bag 38 is not applied to the target tissues as shown in Fig. 15. At this time, the compression bag 38 is exposed in air without touching the biological tissues.

Unlike the case of biological tissues, there is a great difference of acoustic impedance between the compression bag and air, thus high-luminance banded pattern appears as multiple scattering as shown in the B-mode image of Fig. 15 at intervals of integral multiplication of the compression bag film surface distance. However, since the a large reflection occurs at the border between the water and the compression bag 38, the compression bag film surface distance calculating unit 48 can obtain the compression bag film surface distance "d" by setting a proper threshold value.

A border position 111 which is positioned nearest with respect to the central axis of the ultrasonic probe 12 can be obtained as the compression bag film surface distance "d".

The relationship between the pressure inside of the compression bag 38 and the compression bag film surface distance can be obtained in the range of the appropriate compression bag film surface distance for compressing tissues [0, dmax] (dmax is about 1cm) that is set in advance. The solid line in the graph shows the relationship between the pressure and the compression bag film surface distance. Hereinafter the above-mentioned relationship is referred to as the reference condition P0(d), and the operation thereof is referred to as calibration.

The graph in Fig. 16 shows the relationship between the flow volume and the pressure obtained in the condition that the target region is being compressed that is corresponded to the solid line 465 of Fig. 15 showing the relationship between the compression bag film surface distance and the pressure in the condition wherein the compression bag is not applied to the target tissue.

Then diagnosis of tissue elasticity is proceeded wherein the pressure information P(t) and the pressure bag film surface distance information d(t) at an arbitrary time "t" during the compression process of the target tissue is obtained (point "X").

At this time, at the same compression bag film surface distance d(t), amount of inside pressure PO(d(t)) added to the compression bag 38 in the reference condition when the compression bag is not applied to the target tissue can be obtained (point "Y"). The difference from the above-mentioned P(t) of the reference condition is the pressure Ptarget(t) of the compression added to the target tissue at the present time "t", which can be obtained using the following formula 5:

$$\{\text{Formula 5}\} \quad \text{Ptarget}(t) = P(t) - PO(d(t)).$$

While the method for obtaining the compression bag film surface distance "d" in a reception signal of one ultrasonic transducer is described in Fig. 13 - Fig. 16, the present invention does not have to be limited to the above-mentioned method. The compression bag film surface distance "d" may be determined by obtaining the respective compression bag film surface distances "d" using the respective ultrasonic reception signal waveforms received by all ultrasonic transducers provided on the ultrasonic transmitting/receiving surface and calculating the average value thereof as the final compression bag film surface distance.

Also, while the method for detecting the compression bag film surface distance with respect to an RF signal (an ultrasonic reception signal) using a threshold value processing, the similar kind of processing may be executed using a diagnostic image such as B-mode image constructed using RF signals (ultrasonic reception signals).

Also, while the compression bag film surface distance is detected by a method using binarization process for setting a threshold is described, other methods such as the region growing method that is applied widely to contour extraction, etc. or an image processing method such as the pattern matching method applied to image recognition, etc. may be used.

In accordance with the present embodiment, since expansion level of the compression bag 38 is obtained using signal processing, the pressure added to the target tissue can be measured simply and quickly without using the flow sensor 42.

While the previously described embodiment is based on the calibration operation for obtaining the relationship between the reference condition PO(V) by expanding/deflating the compression bag in the condition not being applied to the target tissue, such calibration operation can be omitted by applying the compression bag 38 in which the relationship between the reference condition PO(V) is obtained in advance and storing the obtained relationship in the memory of the pressure calculating unit 46.

Also, the flow sensor unit 42 can calculate and obtain the relationship between the injected flow volume and the compression bag film surface distance by charging/discharging, for example, 5cc of liquid to/from the compression bag 38 in advance using the liquid charging/discharging operation unit 44 and measuring the compression bag film surface distance "d1". In concrete term, by dividing the flow volume of the liquid by the compression bag film surface distance, the flow volume of the liquid to be charged when the compression bag film surface distance shifts by 1mm can be calculated. Then the flow volume of the liquid in accordance with the measured compression bag film surface distance is to be calculated.

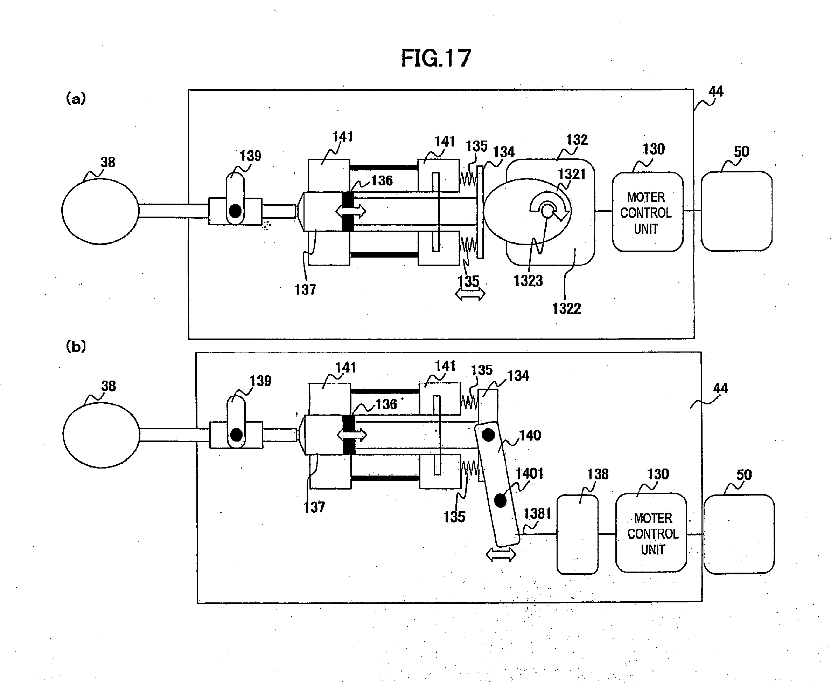

The pattern for compressing the compression bag 38 automatically using the liquid charging/discharging operation unit 44 for applying to the first embodiment and the second embodiment is shown in Fig. 17. The liquid charging/ discharging operation unit 44 shown in Fig. 17(a) is formed by a motor unit 132, a motor control unit 130 for controlling the motor unit 132, a board 134 which moves by the drive of the motor unit 132, a spring 135 for supporting the board 134, a piston 136 which is coupled to the board 134, a cylinder 137 in which the piston 136 is embedded, a cylinder fixing portion 141 for fixing the cylinder 137 and a cock 139 for controlling flow of the liquid. The spring 135 is coupled to the board 134 and the fixing portion 141, and is set so that the board 135 reciprocates by the motor unit 132 and the spring 135.

The motor unit 132 is formed by an elliptic type rotating body 1321 and a motor 1322 for rotating the rotating body 1321. By command of the motor control unit 130, when the motor 1322 is rotated, the elliptical rotating body 1321 rotates while being attached externally to the board 134 centering around the rotation axis 1323. The board 134 reciprocates to the right and the left being pushed toward the left by the rotation of the rotating body 1321 and pushed toward the opposite direction (right direction) of the rotating body 132 by the spring 135.

The piston 136 formed being integrated with the board 134 reciprocates along with reciprocation of the board 134. The liquid in the cylinder 137 is pushed out by the piston 136, and the pushed out liquid reaches the compression bag 38. Then the compression bag 38 is expanded due to the liquid being pushed out.

Stroke for the reciprocating motion of the board 134 is determined based on the position of the rotating axis 1323 and the length of the minor axis and the major axis of the rotating body 1321. For example, when the rotation axis 1323 is placed at the center in the 1323 of the rotating body, the board 134 reciprocates only for the distance of difference between the minor axis and the major axis of the ellipse. Also, when the rotation axis 1323 is placed being shifted from the center of the rotating body 1321, the stroke range varies for the distance that the rotation axis 1323 has been shifted.

In other words, injection amount of the liquid by the piston 136 can be set by the position of the rotation axis 1323 and the length of the minor axis and the major axis of the rotating body 1321 set by the stoke. For example, by using the cross-sectional area of the cylinder 92, it is possible to set the range of the injection amount of the liquid for one stroke for approximately 0.2cc - 1.0cc.

The rotating body can be formed in a variety of shapes, and also can be exchangeable. Also, an encoder is provided to the motor 1322, and the encoder is connected to the image display device 26. The rotation information of the motor 1322 can be displayed on the image display device 26.

Fig. 17(b) shows a pattern of the liquid charging/ discharging operation unit 44 that pushes the board 134 using a wire unit 138. It is formed by the wire unit 138 for driving a wire 1381, a motor control unit 130 for controlling the wire unit 138, a rotating member 140 being connected to the wire 1381 and a board 134 for informing the motion of the wire 1381 to the board 134, the board 134 that reciprocates to the right and the left, a spring 135 for supporting the board 134, a piston 136 to be coupled to the board 134, a cylinder 137 to which the piston 136 is embedded, a cylinder fixing portion 141 for fixing the cylinder 137 and a cock 139 for controlling flow of liquid. The spring 135 is coupled to the board 134 and the fixing portion 141, and the board 134 reciprocates by the wire unit 138 and the spring 135.

A motor for moving the wire 1381 to the right and left directions is incorporated in the wire unit 138, and it can reciprocate the wire 1381 to the right and left directions. The rotating member 140 rotates centering around a center axis 1401. When the wire 1381 is moved to the right side the board 134 is pushed to the left, and when the wire 1381 is moved to the left side the board 134 is pulled to the right.

Transmission of the liquid to the compression bag 38 based on the operation of the board 134 will be omitted since it is similar to the description on Fig. 17(a).

The wire unit 138 is capable of adjusting the stroke width of the wire 1381. For example, it is possible to set the injection amount of the liquid for one stroke in the range of approximately 0.2cc - 1.0cc.

The above-described pattern for automatically compressing the compression bag 38 has the crank mechanism for converting the rotating motion by the motor 1322 or the wire 1381 into the linear motion of the piston 136. It can also have a mechanism for linear motion to make the piston 136 perform linear motion.

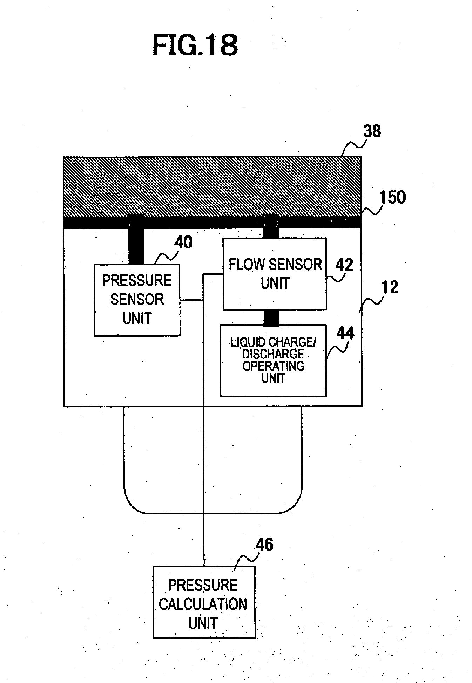

While the previously mentioned pattern had a convex-type probe to insert into a body, the probe may be the linear probe capable of compressing the object 10 from outside of the object 10 as shown in Fig. 18, which can be applied to an arbitrary ultrasonic probe. The compression bag 38 is for compressing the object 10 from outside of the body.

The compression bag 38 is set on the anterior surface of a plurality of transducer elements 150 of the linear-type probe. Inside of the ultrasonic probe 12, the pressure sensor unit 40, the flow sensor unit 42 and the liquid charging/discharging operation unit 44 are comprised. The pressure information detected in the pressure sensor unit 40 and the flow volume information detected in the flow sensor unit 42 are outputted to the pressure calculating unit 46.

First, the pressure sensor 40 measures the pressure in the compression bag 38 in the condition that the compression bag 38 is not applied to the object 10 from outside, and the measured pressure value is stored in the memory in the pressure calculating unit 46 temporally. Then the operator applies and compresses the ultrasonic probe 12 to the object 10. The pressure sensor unit 40 measures the pressure in the compression bag 38 in the condition that the object 10 is being compressed. At this time, volume of the liquid in the compression bag 38 stays constant before and after the compression. The pressure calculating unit 46 calculates the difference between the pressure before compression which is stored in the memory and the pressure after being compressed, and outputs the calculated difference to the elasticity information calculating unit 32 as the pressure of the ultrasonic scanning region of the object 10 to which the compression bag 38 is applied.

## Claims

1. An ultrasonic diagnostic apparatus comprising:

   an ultrasonic probe (12);
   a tomographic image constructing means (20) for constructing a tomographic image based on RF frame data of the tomographic region of an object to be examined (10) via the ultrasonic probe (12);
   an elasticity information calculating means (32) for obtaining strain or elasticity modulus in the tomographic region based on the RF signal frame data;
   an elastic image constructing means (34) for generating an elastic image in the tomographic region based on the strain or the elasticity modulus obtained in the elasticity information calculating means (32);
   a display means for displaying the tomographic image and/or the elastic image; and further comprising
   a compression bag (38) placed on an ultrasonic wave transmitting/receiving surface of the ultrasonic probe (12) in which liquid is to be filled;
   a liquid charging/discharging means (44) for expanding or deflating the compression bag (38) by charging/discharging the liquid to/from the compression bag (38);
   a pressure measuring means (40) for measuring the pressure of the liquid filled in the compression bag (38); and
   a pressure calculating means (46) for calculating the pressure of an ultrasonic scanning region of the object (10) where the compression bag (38) is applied,
   wherein the elasticity information calculating means (32) is configured to calculate elasticity modulus using the calculated pressure information of the pressure calculating means (46),
   **characterized by**
   a flow volume measuring means (42) for measuring the inflow/outflow volume of the liquid in the compression bag (38), wherein
   the pressure calculating means (46) is configured to calculate the pressure of the ultrasonic scanning region of the object (10) to which the compression bag (38) is applied based on the pressure information measured in the pressure measuring means (40) and an inflow/outflow volume of the liquid measured in the flow volume measuring means (42).

2. The ultrasonic diagnostic apparatus according to claim 1, **characterized by** comprising a cock (100) for maintaining volume of the liquid constant in the compression bag (38).

3. The ultrasonic diagnostic apparatus according to claim 1, **characterized in that** the pressure measuring means (40) is configured to obtain the pressure information at a predetermined flow volume.

4. The ultrasonic diagnostic apparatus according to claim 3, **characterized in that** the pressure calculating means (46) is configured to calculate the pressure in the ultrasonic scanning region of the object (10) to which the compression bag (38) is applied based on the difference between a first pressure value measured at the predetermined flow volume by the pressure measuring means (40) in the condition that the compression bag (38) is not applied on the object (10) and a second pressure value measured at the predetermined flow volume in the condition that the compression bag (38) is applied on the object (10).

5. The ultrasonic diagnostic apparatus according to claim 1, wherein the pressure measuring means (40) is embedded in the compression bag (38).

6. The ultrasonic diagnostic apparatus according to claim 1, wherein the pressure measuring means (40) has a film diaphragm (4012) which is configured to be depressed by pressure of the liquid, and a strain gage (4013) for measuring the depression of the film of the diaphragm (4012).

7. The ultrasonic diagnostic apparatus according to claims 1 to 3, wherein the pressure measuring means (40) is placed between the compression bag (38) and the ultrasonic probe (12).

8. The ultrasonic diagnostic apparatus according to claim 1, wherein the pressure measuring means (40) is placed in the liquid charging/discharging means.

9. The ultrasonic diagnostic apparatus according to claim 5, wherein the flow volume measuring means (42) is configured to measure flow volume of the compression bag (38) using the RF signal frame data.

10. The ultrasonic diagnostic apparatus according to claim 9, wherein the flow volume measuring means (42) is configured to measure flow volume of the compression bag (38) by detecting the film surface of the compression bag (38) from the RF signal frame data.

11. The ultrasonic diagnostic apparatus according to claim 1, wherein the liquid charging/discharging means (44) has:

> a main body (80);
> a cylinder (92) filled with liquid inside and fixed to the main body (80);
> a piston (90) and a pusher (88) placed inside of the cylinder (92) for pushing in or pulling out liquid to/from the compression bag (38);
> a pusher fixing unit (86) for fixing the pusher (88); and
> an operation unit (82) for driving the pusher (88).

12. The ultrasonic diagnostic apparatus according to claim 11, wherein:

> a pressure measuring means (40) is comprised in the pusher (88); and
> the pressure measuring means (40) is configured to measure the pressure transmitted from the compression bag (38) by the driving of the pusher (88).

**Patentansprüche**

1. Diagnostische Ultraschallvorrichtung, die umfasst:

> eine Ultraschallsonde (12);
> Mittel (20) zum Erstellen eines Tomographiebildes zum Erstellen eines Tomographiebildes aufgrund von HF-Rahmendaten des Tomographiebereichs eines zu untersuchenden Objekts (10) mit Hilfe der Ultraschallsonde (12);
> Mittel (32) zum Berechnen von Elastizitätsinformationen zum Erhalten des Dehnungskoeffizienten oder des Elastizitätsmoduls in dem Tomographiebereich aufgrund der HF-Signalrahmendaten;
> Mittel (34) zum Erstellen eines elastischen Bildes zum Erzeugen eines elastischen Bildes in dem Tomographiebereich aufgrund des Dehnungskoeffizienten oder des Elastizitätsmoduls, die in den Mitteln (32) für die Berechnung der elastischen Informationen erhalten wurden;
> Anzeigemittel zum Anzeigen des Tomographiebildes und/oder des elastischen Bildes; und ferner umfassend:

>> einen Kompressionsbeutel (38), der auf einer Ultraschallwellensende-/Ultraschallwellenempfangsfläche der Ultraschallsonde (12) positioniert ist und in den Flüssigkeit zu füllen ist;
>> Flüssigkeitseinfüll-/Flüssigkeitsausstoßmittel (44) zum Ausdehnen oder Entleeren des Kompressionsbeutels (38) durch Einfüllen der Flüssigkeit in den bzw. Ausstoßen der Flüssigkeit aus dem Kompressionsbeutel (38);
>> Druckmessmittel (40) zum Messen des Drucks der in den Kompressionsbeutel eingefüllten Flüssigkeit; und
>> Druckberechnungsmittel (46) zum Berechnen des Drucks eines Ultraschallabtastbereichs des Objekts (10), wo der Kompressionsbeutel (38) angewendet wird,
wobei die Mittel (32) zum Berechnen der Elastizitätsinformationen konfiguriert sind, den Elastizitätsmodul unter Verwendung der berechneten Druckinformationen der Druckberechnungsmittel (46) zu berechnen, **gekennzeichnet durch**
Mittel (42) zum Messen der Durchflussmenge zum Messen der Zufluss-/Abflussmenge der Flüssigkeit in den bzw, aus dem Kompressionsbeutel (38), wobei
die Druckberechnungsmittel (46) konfiguriert sind, den Druck des Ultraschallabtastbereichs des Objektes (10), auf das der Kompressionsbeutel (38) angewendet wird, anhand der in den Druckmessmitteln (40) gemessenen Druckinformationen und einer in den Durchfiussengenmessmitteln (42) gemessenen Zufluss-/Abflussmenge der Flüssigkeit zu berechnen.

2. Diagnostische Ultraschallvorrichtung nach Anspruch 1, **dadurch gekennzeichnet, dass** sie ein Hahnventil (100) umfasst, um das Volumen der Flüssigkeit in dem Kompressionsbeutel (38) konstant zu halten.

3. Diagnostische Ultraschallvorrichtung nach Anspruch 1, **dadurch gekennzeichnet, dass** die Druckmessmittel (40) konfiguriert sind, die Druckinformationen bei einer vorgegebenen Durchflussmenge zu erhalten.

4. Diagnostische Ultraschallvorrichtung nach Anspruch 3, **dadurch gekennzeichnet, dass** die Druckberechnungsmittel (46) konfiguriert sind, den Druck in dem Ultraschallabtastbereich des Objektes (10), auf das der Kompressionsbeutel (38) angewendet wird, anhand des Unterschieds zwischen einem ersten bei der vorgegebenen Durchflussmenge durch die Druckmessmittel (40) unter der Bedingung, dass der Kompressionsbeutel (38) nicht auf das Objekt (10) angewendet wird, gemessenen Druckwert und einem zweiten bei der vorgegebenen Durchflussmenge unter der Bedingung, dass der Kompressionsbeutel (38) auf das Objekt (10) angewendet wird, gemessenen Druckwert zu berechnen.

5. Diagnostische Ultraschallvorrichtung nach Anspruch 1, wobei die Druckmessmittel (40) in den Kompressionsbeutel eingebettet sind.

6. Diagnostische Ultraschallvorrichtung nach Anspruch 1, wobei die Druckmessmittel (40) ein Schichtdiaphragma (4012), das konfiguriert ist, durch den Druck der Flüssigkeit heruntergedrückt zu werden, und einen Dehnungsmessstreifen (4013) zum Messen des Herunterdrückens der Schicht des Diaphragmas (4012) besitzen.

7. Diagnostische Ultraschallvorrichtung nach den Ansprüchen 1 bis 3, wobei die Druckmessmittel (40) zwischen dem Kompressionsbeutel (38) und der Ultraschallsonde (12) positioniert sind.

8. Diagnostische Ultraschallvorrichtung nach Anspruch 1, wobei die Druckmessmittel (40) in den Flüssigkeitseinfüll-/Flüssigkeitsausstoßmitteln positioniert sind.

9. Diagnostische Ultraschallvorrichtung nach Anspruch 5, wobei die Durchflussmengenmessmittel (42) konfiguriert sind, die Durchflussmenge des Kompressionsbeutels (38) unter Verwendung der HF-Signalrahmendaten zu messen.

10. Diagnostische Ultraschallvorrichtung nach Anspruch 9, wobei die Durchflussmengenmessmittel (42) konfiguriert sind, die Durchflussmenge des Kompressionsbeutels (38) durch Detektieren der Schichtfläche des Kompressionsbeutels (38) aus den HF-Signalrahmendaten zu messen.

11. Diagnostische Ultraschallvorrichtung nach Anspruch 1, wobei die Flüssigkeitseinfüll-/Flüssigkeitsausstoßmittel (44) umfassen:

einen Hauptkörper (80);
einen Zylinder (92), der ihnen mit Flüssigkeit gefüllt ist und an dem Hauptkörper (80) befestigt ist;
einen Kolben (90) und einen Stößel (88), die innerhalb des Zylinders (92) zum Hineindrücken oder Herausziehen der Flüssigkeit in den/aus dem Kompressionsbeutel (38) positioniert sind;
eine Stößelbefestigungseinheit (86) zum Befestigen des Stößels (88); und
eine Betriebseinheit (82) zum Antreiben des Stößels.

12. Diagnostische Ultraschallvorrichtung nach Anspruch 11, wobei:

Druckmessmittel (40) in dem Stößel (88) enthalten sind; und

die Druckmessmittel (40) konfiguriert sind, den von dem Kompressionsbeutel (38) durch das Antreiben des Stößels (88) übertragenen Druck zu messen.

**Revendications**

1. Appareil de diagnostic par ultrasons, comprenant :

    une sonde à ultrasons (12) ;
    un moyen de construction d'une image tomographique (20) pour construire une image tomographique sur la base de données de trame RF de la région tomographique d'un objet à examiner (10) via la sonde à ultrasons (12) ;
    un moyen de calcul d'information d'élasticité (32) pour obtenir un module de contrainte ou un module d'élasticité dans la région tomographique sur la base des données de trame de signal RF;
    un moyen de construction d'image élastique (34) pour générer une image élastique dans la région tomographique en se basant sur le module de contrainte ou le module d'élasticité obtenu dans le moyen de calcul d'information d'élasticité (32) ;
    un moyen d'affichage pour afficher l'image tomographique et/ou l'image élastique ; et comprenant en outre
    un sac à compression (38) placé sur une surface de transmission/réception d'onde ultrasonore de la sonde à ultrasons (12) dans lequel un liquide doit être rempli ;
    un moyen de charge/décharge de liquide (44) pour gonfler ou dégonfler le sac à compression (38) en chargeant/déchargeant le liquide vers/depuis le sac à compression (38) ;
    un moyen de mesure de pression (40) pour mesurer la pression du liquide rempli dans le sac à compression (38) ; et
    un moyen de calcul de pression (46) pour calculer la pression d'une région de scannage par ultrasons de l'objet (10) dans laquelle le sac à compression (38) est appliqué,
    dans lequel le moyen de calcul d'information d'élasticité (32) est configuré pour calculer le module d'élasticité en utilisant l'information-de pression calculée du moyen de calcul de pression (46),
    **caractérisé par**
    un moyen de mesure de volume écoulé (42) pour mesurer le volume entrant/sortant du liquide dans le sac à compression (38), dans lequel le moyen de calcul de pression (46) et configuré pour calculer la pression de la région de scannage par ultrasons de l'objet (10) sur laquelle le sac à compression (38) est appliqué, en se basant sur l'information de pression mesurée dans le moyen de mesure de pression (40) et sur un volume entrant/sortant du liquide mesuré dans le moyen de mesure de volume écoulé (42).

2. Appareil de diagnostic par ultrasons selon la revendication 1, **caractérisé en ce qu'**il comprend un robinet (100) pour maintenir constant le volume de liquide dans le sac à compression (48).

3. Appareil de diagnostic par ultrasons selon la revendication 1, **caractérisé en ce que** le moyen de mesure de pression (40) est configuré pour obtenir l'information de pression à un volume écoulé prédéterminé.

4. Appareil de diagnostic par ultrasons selon la revendication 3, **caractérisé en ce que** le moyen de calcul de pression (46) est configuré pour calculer la pression dans la région de scannage par ultrasons de l'objet (10), dans laquelle le sac à compression (38) est appliqué, en se basant sur la différence entre une première valeur de pression mesurée au volume écoulé prédéterminé, par le moyen de mesure de pression (40), dans la condition dans laquelle le sac à compression (38) n'est pas appliqué sur l'objet (10), et une seconde valeur de pression mesurée au volume écoulé prédéterminé, dans la condition dans laquelle le sac à compression (38) est appliqué sur l'objet (10).

5. Appareil de diagnostic par ultrasons selon la revendication 1, dans lequel le moyen de mesure de pression (40) est noyé dans le sac à compression (38).

6. Appareil de diagnostic par ultrasons selon la revendication 1, dans lequel le moyen de mesure de pression (40) comprend un diaphragme en film (4012) qui est configuré pour être enfoncé par la pression du liquide, et une jauge de contrainte (4013) pour mesurer l'enfoncement du film du diaphragme (4012).

7. Appareil de diagnostic par ultrasons selon les revendications 1 à 3, dans lequel le moyen de mesure de pression (40) est placé entre le sac à compression (38) et la sonde à ultrasons (12).

**8.** Appareil de diagnostic par ultrasons selon la revendication 1, dans lequel le moyen de mesure de pression (40) est placé dans le moyen de charge/décharge de liquide.

**9.** Appareil de diagnostic par ultrasons selon la revendication 5, dans lequel le moyen de mesure de volume écoulé (42) est configuré pour mesurer le volume écoulé du sac à compression (38) en utilisant les données de trame de signal RF.

**10.** Appareil de diagnostic par ultrasons selon la revendication, dans lequel le moyen de mesure de volume écoulé (42) est configuré pour mesurer le volume écoulé du sac à compression (38) en détectant la surface du film de sac à compression (38) à partir des données de trame de signal RF.

**11.** Appareil de diagnostic par ultrasons selon la revendication 1, dans lequel le moyen de charge/décharge de liquide (44) comprend :

un corps principal (80) ;
un cylindre (92) rempli de liquide à l'intérieur et fixé sur le corps principal (80) ;
un piston (90) et un poussoir (80) placé à l'intérieur du cylindre (92) pour pousser du liquide vers l'intérieur du sac à compression (38) ou pour tirer du liquide hors du sac à compression (38) ;
une unité de fixation de poussoir (86) pour fixer le poussoir (88) ; et
une unité d'actionnement (82) pour entraîner le poussoir (88).

**12.** Appareil de diagnostic par ultrasons selon la revendication 11, dans lequel :

un moyen de mesure de pression (40) est compris dans le poussoir (88) ; et
le moyen de mesure de pression (40) est configuré pour mesurer la pression transmise depuis le sac à compression (38) par l'entraînement du poussoir (88).

# FIG.1

1

TRANSMISS-ION UNIT — 14

TRANSMITTION/ RECEPTION CONTROL UNIT — 17

RECEPTION UNIT — 16

PHASING ADDITION UNIT — 18

TOMOGRAPHIC IMAGE CONSTRUCTION UNIT — 20

BLACK AND WHITE SCAN CONVERTER — 22

SWITCHING ADDITION UNIT — 24

IMAGE DISPLAY — 26

RF SIGNAL FRAME DATA STORING UNIT — 28

DISPLACEMENT MEASURING UNIT — 30

ELASTICITY INFORMAITON CALCULATING UNIT — 32

ELASTIC IMAGE CONSTR-UCTING UNIT — 34

COLOR SCAN CONVERTER — 36

BAG FILM SURFACE DISTANCE CALCULATING UNIT — 48

PROBE — 12

PRESSURE SENSOR UNIT — 40

PRESSURE CALCULATION UNIT — 46

PRESSURE BAG — 38

OBJECT — 10

FLOW SENSOR UNIT — 42

LIQUID CHARGE/DISCHARGE OPERATING UNIT — 44

APPARATUS CONTROL INTERFACE UNIT — 50

EP 2 008 591 B1

# FIG.2

(a)

INSERTION
SECTION

PROBE
GRIP
SECTION

(b)

LIUQID
CHAGE/DISCHARGE
OPERATING UNIT

(c)

# FIG.3

EP 2 008 591 B1

# FIG.4

PRESSURE SENSOR UNIT **40**

P(t)

PRESSURE CALCULATION UNIT **46**

TO PRESSURE INFORMATION CALCULATING UNIT 32

**38**

**37**

**100**

LIQUID CHARGE/DISCHARGE OPERATING UNIT **44**

**60** **38** **39**

EP 2 008 591 B1

# FIG.5

FRONT VIEW          CROSS-SECTION

(a)

38

38

62

60

60

VOLUME : V0
PRESSURE : P0

(b)

TARGET TISSUE                TARGET TISSUE

38

38

62

60

60

VOLUME : V0
PRESSURE : P(t)

# FIG.6

PRESSURE SENSOR UNIT **40**

P(t)

PRESSURE CALCULATION UNIT **46**

V(t)

**38**

**37**

FLOW SENSOR UNIT **42**

LIQUID CHARGE/DISCHARGE OPERATING UNIT **44**

**60** **38** **39**

EP 2 008 591 B1

# FIG.7

# FIG.8

TARGET TISSUE

38

37

40
PRESSURE SENSOR UNIT

P(t)

V(t)

42
FLOW SENSOR UNIT

44
LIQUID CHARGE/DISCHARGE OPERATING UNIT

46
PRESSURE CALCULATION UNIT

PRESSURE P [kPa]

P0(V) CURVE

463

P(t)       X

462       461

P0(V(t))       Y

0       V(t)       Vmax

FLOW VOLUME V [cc]

# FIG.9

(a)

PRESSURE SENSOR UNIT — 40

P(t)

PRESSURE CALCULATION UNIT — 46

V(t)

FLOW SENSOR UNIT — 42

LIQUID CHARGE/DISCHARGE OPERATING UNIT — 44

38

37

401

402

(b)

PRESSURE

401

4011

4012

4011

4013

402

60

38

39

401

EP 2 008 591 B1

# FIG.10

PRESSURE SENSOR SIGNAL LINE

PRESSURE SENSOR SIGNAL LINE

EP 2 008 591 B1

# FIG.11

EP 2 008 591 B1

# FIG.12

EP 2 008 591 B1

# FIG.13

EP 2 008 591 B1

# FIG.14

# FIG.15

FREE CONDITION

ULTRASNOIC
WAVE
TRANSMITTING
/RECEIVING
SURFACE

38

CENTRAL AXIS

40

PRESSURE
SENSOR
UNIT

P

44

LIQUID CHARGE/
DISCHARGE
OPERATING UNIT

d

46

PRESSURE CALCULATING UNIT

P0(d) CURVE

dmax

0

COMPRESSION BAG FILM
SURFACE DISTANCE d [mm]

PRESSURE P [kPa]

B-MODE IMAGE

MULTIPLE ECHO SIGNALS OF
THE PRESSURE BAG BORDER

ECHO SIGNAL OF
THE PRESSURE
BAG BORDER

48

BAG FILM
SURFACE
DISTANCE
CALCULATING
UNIT

d

CENTRAL AXIS

EP 2 008 591 B1

**FIG.16**

# FIG.17

(a)

(b)

MOTER CONTROL UNIT

EP 2 008 591 B1

34

# FIG.18

PRESSURE
SENSOR
UNIT
40

FLOW SENSOR
UNIT
42

LIQUID CHARGE/
DISCHARGE
OPERATING
UNIT
44

38

150

12

PRESSURE
CALCULATION
UNIT
46

EP 2 008 591 B1

**EP 2 008 591 B1**

**REFERENCES CITED IN THE DESCRIPTION**

**Patent documents cited in the description**

- JP 2004267464 A
- EP 1629777 A1
- US 2004210136 A1
- US 5265612 A